# EUROPEAN PATENT APPLICATION

(11) **EP 2 360 147 A1**
(43) Date of publication of application: **24.08.2011**
(21) Application number: 10154258.7
(22) Date of filing: 22.02.2010
(51) Int. Cl.: C07D 215/26, A61K 31/4704

(54) **Process for preparing crystalline particles of a salt of 8-hydroxy-5-[(1R)-1-hydroxy-2-[[(1R)-2-(4-methoxyphenyl)-1-methylethyl]amino] ethyl]-2(1H)-quinolinone (carmoterol)**

(71) Applicant: CHIESI FARMACEUTICI S.p.A., 43100 Parma (IT)
(72) Inventor: COCCONI, Daniela, 43100, PARMA (IT); MUSA, Rossella, 43100, PARMA (IT); BRAMBILLA, Gaetano, 43100, PARMA (IT); PARIKH, Dipesh, 43100, PARMA (IT); RUECROFT, Graham, 43100, PARMA (IT)
(74) Representative: Minoja, Fabrizio

(57) **Abstract**

The present invention relates to a process for preparing crystalline particles of a pharmaceutically acceptable salt of 8-hydroxy-5-[(1R)-1-hydroxy-2-[[(1R)-2-(4-methoxyphenyl)-1-methylethyl]amino]ethyl]-2(1H)-quinolinone (carmoterol) and to the particles as obtained by said process.

## Description

### FIELD OF INVENTION

The present invention relates to a process for preparing crystalline particles of a pharmaceutically acceptable salt of 8-hydroxy-5-[(1R)-1-hydroxy-2-[[(1R)-2-(4-methoxyphenyl)-1-methylethyl]amino]ethyl]-2(1H)-quinolinone (carmoterol).

The invention also relates to particles as obtained by said process.

### BACKGROUND OF THE INVENTION

The pharmaceutically active compound 8-hydroxy-5-[(1 R)-1-hydroxy-2-[[(1R)-2-(4-methoxyphenyl)-1-methylethyl]amino]ethyl]2(1H)-quinolinone (I), also known as carmoterol, has been described in EP 147719 as a bronchodilator having a potent beta₂-adrenoceptor stimulating action

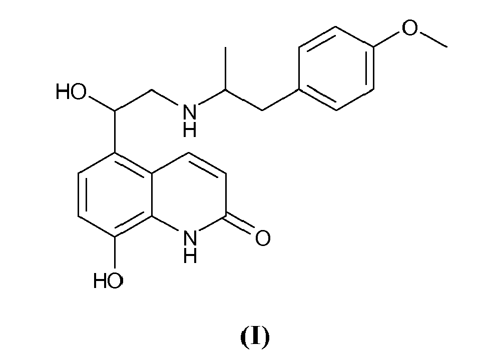

In the art, its monohydrochloride salt has been widely investigated as a medicament for the treatment of respiratory disorders such as asthma and chronic obstructive pulmonary disease (COPD), and has been referred to with the code TA 2005 or CHF 4226.

However, so far, the monohydrochloride salt can be obtained in an adequate pharmaceutical level of chemical purity and crystallinity, only by applying specific crystallization conditions, as reported in WO 2005/089760.

Moreover said salt is presently under development as formulations for inhalation at a very low single dose, ranging from 1 to 4 g per actuation of the inhaler.

Therefore, in the case of powder or suspension formulations, it is important that the drug disperses well and is uniformly distributed in the composition as a lack of homogeneity, upon administration through the inhaler, could give rise to a risk of over- or under-dosage, and hence is detrimental to the possibility of achieving a reproducible accuracy of the delivered dose.

In view of the aforementioned problems, it would be advantageous to provide a carmoterol salt which is easy to obtain in a highly crystalline form and has good handling qualities for pharmaceutical use, in particular in terms of dispersibility in a composition for inhalation.

It has now been found a process for the preparation of salts of carmoterol having a very high degree of crystallinity and good physico-chemical properties so that they can be formulated as dry powder or suspension formulations for inhalation with a good homogeneity.

### SUMMARY OF THE INVENTION

The present invention is directed to a process for preparing crystalline particles of a pharmaceutically acceptable salt of 8-hydroxy-5-[(1R)-1-hydroxy-2-[[(1R)-2-(4-methoxyphenyl)-1-methylethyl] amino]ethyl]-2(1H)-quinolinone (carmoterol), said process comprising the steps of a) preparing a solution of said active ingredient in a pre-determined ratio in a solvent; b) contacting said solution in a first flowing stream with a non-solvent in a second flowing stream so as to form an emulsion; c) applying high intensity ultrasound to induce crystallisation of the active ingredient; and d) isolating and collecting the active ingredient particles, wherein the solvent is a methanol:ethanol mixture in a ratio between 4:1 and 2:1 (v/v), preferably 3:1, and the anti-solvent is n-heptane.

The invention is also directed to crystalline particles of a pharmaceutically acceptable salt of carmoterol obtained by the aforementioned process.

In another aspect, the present invention provides a formulation for administration by inhalation comprising the aforementioned particles, optionally together with one or more pharmaceutically acceptable excipients.

Preferably, the formulation is provided in the form of dry inhalation powder to be used with dry powder inhaler (DPI) devices or in the form of a suspension of the particles in a propellant gas to be used with pressurized metered-dose inhaler (pMDI) devices.

Therefore the invention is also directed to a device which may be a single- or multi-dose dry powder inhaler, or a pressurized metered dose inhaler, respectively, filled with the aforementioned formulations.

### DEFINITIONS

In the disclosure, the following terms have the meanings specified hereinbelow:

"Drug", "active ingredient", "active agent" and "active substance" are used as synonyms.

"Solvent" means the medium in which the active ingredient is dissolved and "anti-solvent" means the medium in which its crystallization takes place.

"Very low-dosage strength" means active ingredients endowed with particularly high potency which are present in the powder formulation in a very low concentration. Said active ingredients are commonly administered at a daily therapeutic dose lower than 6 µg.

"Daily therapeutically effective dose" means the quantity of active ingredient administered in one day by inhalation. Said daily dose may be delivered in one or more administrations per day and in one or more actuations of the inhaler per administration.

"Actuation" means the release of active ingredient from the device by a single activation (e.g. mechanical or breath).

The daily dose may be reached by a single or double administration.

In an embodiment the daily dose may be reached by a single administration and delivered in one actuation of the inhaler.

In another preferred embodiment the daily dose may be reached by a single administration and delivered in more actuations of the inhaler, preferably two.

In a further embodiment the daily dose may be reached by a double administration and delivered in one actuation of the inhaler.

In still a further embodiment the daily dose may be reached by a double administration and delivered in more actuations of the inhaler, preferably two.

"Very high degree of crystallinity" means a degree, expressed as weight percent of the crystalline compound with respect to the total weight of the compound, of at least 97%, preferably higher than 98%, more preferably equal to or higher than 99%.

In this context, the particle size refers to the dimension of the particles and is quantified by measuring a characteristic equivalent sphere diameter, known as volume diameter, by laser diffraction.

The particle size may also be quantified by measuring the mass diameter by means of suitable instrument well known to the skilled person such as, for instance, the sieve analyser.

The volume diameter (VD) is related to the mass diameter (MD) by the density of the particles (assuming a size independent density for the particles).

In the present application, the particle size is expressed in terms of volume diameter and the particle size distribution is expressed in terms of: i) the median volume diameter (MVD) which corresponds to the diameter of 50 percent by weight or volume respectively [d(v,0.5)], of the particles, and ii) the MD in micron of 10% and 90% of the particles, respectively [d(v,0.1) and d(v,0.9)].

Upon aerosolisation, the particle size is expressed as mass aerodynamic diameter (MAD) which indicates the capability of the particles of being transported and suspended in an air stream. The term MMAD stands for median mass aerodynamic diameter.

As used herein, the expression "good homogeneity" refers to a formulation wherein, upon mixing, the content uniformity of the active ingredient, expressed as relative standard deviation (RSD), is less than 5%.

As used herein, the expression 'respirable fraction' refers to an index of the percentage of active particles which would reach the deep lungs in a patient.

The respirable fraction, also termed fine particle fraction, is evaluated using suitable *in vitro* apparata such as the Andersen Cascade Impactor (ACI) or the Multi Stage Liquid Impinger (MLSI) according to procedures reported in common Pharmacopoeias. It is calculated by the ratio between the respirable dose and the delivered dose. The delivered dose is calculated from the cumulative deposition in the apparatus, while the respirable dose (fine particle dose) is calculated from the deposition on Stages 3 (S3) to filter (AF) corresponding to particles ≤ 5.0 micron.

As used herein the term "interactive or ordered mixture" refers to powder formulation for inhalation comprising a pharmacologically-inert physiologically acceptable carrier substance, to which the micronised active compound particles are bonded by adhesion in order thus to achieve and to maintain a suitable mixed material, i.e. homogeneity of the mixture.

As used herein, the term "relatively highly fissured surface" means a surface on which there are clefts and valleys and other recessed regions, referred to herein collectively as fissures. Said surface of the coarse excipient particles may be defined in terms of fissure index or rugosity coefficients as disclosed in WO 01/78695 and WO 01/78693 and they can be characterized according to the description therein reported.

### FIGURES

Figure 1 - SEM image of the particles obtained in Example 1
Figure 2 - Thermogram of the particles obtained in Example 1

### DETAILED DESCRIPTION OF THE INVENTION

The present invention is directed to a process for preparing crystalline particles of a pharmaceutically acceptable salt of 8-hydroxy-5-[(1R)-1-hydroxy-2-[[(1 R)-2-(4-methoxyphenyl)-1-methylethyl]amino]ethyl]-2(1H)-quinolinone (carmoterol), said process comprising the steps of:
a) preparing a solution of said active ingredient in a pre-determined ratio in a solvent;
b) contacting said solution in a first flowing stream with a non-solvent in a second flowing stream so as to form an emulsion;
c) applying high intensity ultrasound to induce crystallisation of the active ingredient; and
d) isolating and collecting the active ingredient particles,
wherein the solvent is a methanol:ethanol mixture in a ratio between 4:1 and 2:1 (v/v), preferably 3:1, and the anti-solvent is n-heptane.

Examples of carmoterol pharmaceutically acceptable salts include hydrochloride, hydrobromide, sulphate, phosphate, maleate, fumarate, tartrate, citrate, benzoate, mesylate, ascorbate, salicylate, acetate, succinate, lactate, glutarate or gluconate.

Preferably carmoterol is used in the form of hydrochloride salt.

As for step a), by varying the concentration of the active ingredient in the solution, it is possible to affect the resultant particle size distribution.

For obtaining particles having a MVD of 3-6 micron, it is preferable to utilise a concentration of active ingredient higher than 1.0% w/v, preferably comprised between 1.0 and 3.0% w/v, more preferably of 1.5% w/v.

In step b), the solution is prepared at a temperature higher than the temperature of the non-solvent, preferably at room temperature, thereby generating a supersaturation when is contacted with the n-heptane (non-solvent), preferably, kept at 5°C.

The temperature of the obtained emulsion is then slowly raised, typically to 50°C to remove methanol from the emulsified droplets.

It is important to consider how the emulsion is prepared in the first instance. The most important parameters to consider include flow rate of solute solution, recirculation flow-rate of non-solvent, the stirring rate, and intensity of applied ultrasound. The addition of emulsifying agents or stabilizers can also assist in obtaining a stable emulsion.

The flow rate ratio of non-solvent to solvent also affects the resultant particle size distribution. In order to prepare particles having a MVD of 3-6 micron, it is preferable to utilise flow rate ratios ranging from 1:1 to 1000:1, preferably from 400:1 to 20:1.

The flowing stream of the solution and the flowing stream of the non-solvent may be contacted or mixed together such that the two streams flow along a single path or axis in the same direction, for example, within the lumen of a suitable delivery means and into a suitable receptacle or chamber, such as an ultrasonic continuous flow cell. Each of the said flowing streams may be pumped at a pre-determined rate of flow from an initial source reservoir into the delivery means. A suitable delivery means may comprise a tubular means such as a straight or curved conduit, for example a pipe, and the two streams may be mixed coaxially therein. Alternatively, the two streams may be introduced into a receptacle or chamber, such as an ultrasonic continuous flow cell, via pumping through separate delivery means, such as two separate tubular means, for example, two pipes.

Typically, the flow rate of the non-solvent stream through an apparatus suitable for producing crystalline particles using the process of the invention is in the range of litres per hour (L/hr) [e.g. 20 L/hr] rather than millilitres per hour (ml/hr). For example, the flow rate for the non-solvent stream flow may be 20 L/hr and that of the first stream 60 ml/hr for a bench top apparatus. Where the process is employed in a larger apparatus, for example, a 100 litre (100 L) vessel the flow rate for the non-solvent stream may be 2400 L/hr and for the solution 120 L/hr. Naturally, the man skilled in the art will appreciate that the rate of flow for each of the said streams can be at any desired speed provided that the flow rate ratio of the two streams is that described for the present invention.

The flow rate of the non-solvent for a small scale apparatus, such as one having a 1 litre capacity, 5 litres or 10 litres capacity, may be typically between 4 and 50 L/hr.

Correspondingly, the flow rate of the added solution can be the same or less than that of the non- solvent, for example, at least 20 times less than that of the non-solvent.

Formation of the emulsion may be carried out in the vessel in which crystallisation takes place, or can be carried out in a separate vessel which is preferably equipped with means for high flow throughput of the non-solvent or it may be equipped with mixing, high shear equipment and/or heating means.

In step d) ultrasonic energy is applied to the emulsion so as to induce nucleation and subsequent crystallisation droplets thus generating the crystalline particles of the salt of carmoterol.

The ultrasonic energy may be applied continuously or in a discontinuous manner such as by pulsed application. Any suitable source of ultrasonic vibration may be used. An ultrasonic probe may, for example, be inserted into the collection vessel, an ultrasonic emitter may be contained in the collection vessel or the collection vessel may be housed in an ultrasonic bath.

The amplitude and frequency of the ultrasound waves affects the rate of nucleation and crystal growth. The frequency of the ultrasound waves may for example range from 20 kHz to 1 MHz, preferably from 10-500 kHz, more preferably from 10 - 100 kHz such as at 10, at 20, 40, 60, 80, or 100 kHz or at any frequency therein between, such as, 30 kHz or 50 kHz.

The ultrasonic irradiation is employed at an amplitude that is appropriate for the formation of crystals of the desired size. For laboratory probe systems with an emitting face of, for example 80 cm², the amplitude selected may be from about 1 - 30 µm, typically from 3 to 20 µm, preferably from 5 to 10 µm.

Probes having a probe face surface area of 8² cm and a power requirement of from 5-80 W, provide a power density of from 0.6 - 12.5 W/cm² using an amplitude of 2-15 mm. In larger systems, comprising transducers bonded onto the flow cell, for example a 6 litre flow cell, the power density for the transducers employed may be from 10 - 100 W/L, preferably from 30-80 W/L, and more preferably from 50-75 W/L, for example 60 W/L or 70 W/L.

For example, an ultrasonic probe operating at the frequency of 20 kHz and at a power of 30-40 W may be advantageously used.

Isolation of crystals from the emulsion can be carried out by any conventional means, such as by filtration, centrifugation, spray-drying, supercritical carbon dioxide evaporation or evaporation.

The process may be carried out in conventional reactors employed in the art such as in a batch fed reactor or in a continuous flow reactor, depending on design. The man skilled in the art is well acquainted with such reactor types and their operation.

Typical apparatus are reported in the co-pending applications n. WO 2008/155570 and n. WO 2008/114052.

Other process parameters may be suitably adjusted according to the teaching of WO 2008/155570 and WO 2008/114052.

The process of the present invention provides microparticles in form of needles, as it can be appreciated by scanning electron microscopy (SEM), and having a very high degree of cristallinity as determined by methods known to the skilled person such as microcalorimetry or differential scanning calorimetry.

Surprisingly, the particles obtained by the process of the invention exhibit excellent dispersion properties allowing to easily obtaining homogenous formulations, in particular when the particles are formulated as dry powders for inhalation.

The high degree of crystallinity and the habit form may explain their good dispersion properties.

The particles are substantially in a pure form, e.g. of at least of 95% w/w, preferably 98% or 99% w/w or even greater.

The chemical purity may be determined according to methods known to the skilled person such as high-performance liquid chromatography (HPLC).

The particles obtained by the process of the invention have a narrow particle size distribution in a range suitable for their intended administration by inhalation.

Advantageously the particles of the invention have a particle size distribution wherein at least 90% of the particles have a diameter equal to or lower than 18 micron, preferably lower than 15 micron, as determined by measuring the characteristic equivalent sphere diameter, known as volume diameter, by laser diffraction as described above, preferably using a Malvern or an equivalent apparatus.

Preferably no more than 10% of said particles have a volume diameter [d(v,0.1)] lower than 2.0 micron, no more than 50% of have a volume diameter [d(v,0.5)] lower than 2.0 micron, preferably comprised between 3 and 6 micron.

In another aspect the present invention provides a formulation for administration by inhalation comprising the particles of the invention. The particles may be formulated together with one or more pharmaceutically acceptable excipients, additives, diluents or carriers.

For example, the formulation is provided in the form of suspension in a propellant as aerosol carrier to be administered by pressurized meted dose inhalers (pMDI).

The pMDI comprises a canister wherein the formulation is filled and a metering valve for delivering a daily therapeutically effective dose of the formulation.

In certain embodiments the aerosol carrier may consist of a non-chlorofluorocarbon-based propellant such as hydrofluoralkane (HFA). In particular the propellants HFA 134a, and HFA 227 or mixtures thereof may be advantageously used.

The suspension formulation may comprise additional excipients such as surfactants, and wetting agents.

In a preferred embodiment, the formulation is provided in the form of dry powder for inhalation, more preferably in the form of an interactive or ordered mixture, by diluting the particles of the invention in a pharmacologically inert physiologically acceptable excipient consisting of coarser particles.

Advantageously, said powder formulation for inhalation may comprise the particles according to the invention and coarse particles of a physiologically acceptable excipient, e.g. particles having a MMD higher than 90 micron and preferably the MD comprised between 50 micron and 500 micron, more preferably between 150 and 400 micron, even more preferably between 210 and 355 micron. In another embodiment, the coarse particles have a MD comprised between 90 and 150 micron.

In one of the preferred embodiments, when their MD is comprised between 210 and 355 micron, the coarse excipient particles have preferably a relatively highly fissured surface.

Preferably the relevant powder formulation may further comprise a fraction of pharmacologically-inert microparticles having a MMD lower than 35 micron composed of particles of a physiologically acceptable excipient and an additive material selected from the class of the anti-adherents such as the amino acids leucine and isoleucine or of the lubricants such as magnesium stearate; sodium stearyl fumarate stearyl alcohol, stearic acid and sucrose monopalmitate.

More preferably said powder formulation comprises a fraction of said pharmacologically-inert microparticles having a MMD lower than 15 micron, preferably lower than 10 micron, composed of particles of a physiologically acceptable excipient and particles of magnesium stearate according to the teaching of EP 1274406.

In another preferred embodiment of the invention, when their MD is comprised between 90 and 150 micron, the coarse carrier particles have preferably a surface rugosity expressed as the fractal dimension of less than or equal to 1.1, determined according to the teaching of EP 1196146. More preferably the surface of said particles is coated with magnesium stearate.

Magnesium stearate is added to the formulations herein described with the aim of improving the respirable fraction of the active substance.

The physiologically acceptable excipient may be any amorphous or crystalline physiologically acceptable pharmacologically inert material of animal or vegetal source or combination thereof. Preferred materials are crystalline sugars and for example monosaccharides such as glucose or arabinose, or disaccharides such as maltose, saccharose, dextrose or lactose. Polyalcohols such as mannitol, sorbitol, maltitol, lactitol may also be used. The most preferred material is α-lactose monohydrate.

Examples of commercial lactose are Capsulac^{™} and Pharmatose^{™}. An example of commercial mannitol is Pearlitol^{™}.

In a preferred embodiment, the fraction of microparticles is composed of 98% by weight of α-lactose monohydrate and 2% by weight of magnesium stearate and the ratio between the fraction of microparticles and the fraction of coarse particles made of α-lactose monohydrate particles is 10:90% by weight, respectively.

The amount of magnesium stearate in the final formulation is advantageously comprised between 0.02% and 1.0% by weight on the total weight of the formulation, preferably between 0.05 and 0.5% by weight, more preferably between 0.1 and 0.4% by weight, even more preferably between 0.2 and 0.3% by weight.

The powder formulation for inhalation comprising the powder particles according to the invention is characterized by a high degree of homogeneity. After the mixing, the content uniformity of the active ingredient, expressed as relative standard deviation (RSD), is less than 5%, preferably equal to or less than 3.5%, more preferably equal to or less than 1.5%.

Said powder formulation may be administered by inhalation with any type of DPIs known in the art.

DPIs can be divided into two basic types: i) single dose inhalers, for the administration of pre-subdivided single doses of the active compound; ii) multidose dry powder inhalers (MDPIs), either with pre-subdivided single doses or pre-loaded with quantities of active ingredient sufficient for multiple doses. On the basis of the required inspiratory flow rates (1/min) which in turn are strictly depending on their design and mechanical features, DPIs are divided in: i) low-resistance devices (> 90 1/min); ii) medium-resistance devices (about 60 1/min); iii) high-resistance devices (about 30 1/min).

The particles of the invention are indicated for the prevention and/or treatment of inflammatory or obstructive airways diseases such as asthma and chronic obstructive pulmonary disease (COPD).

Other respiratory disorders characterised by obstruction of the peripheral airways as a result of inflammation and/or presence of mucus such as chronic obstructive bronchiolitis, bronchiectasies, and cystic fibrosis may also benefit by their use.

The invention is further illustrated by the following examples.

### EXAMPLES

### Example 1 - Preparation of crystalline particles of carmoterol hydrochloride

The particles were prepared according to the teachings of WO 2008/155570 and WO 2008/114052.

n-Heptane was charged to a crystallizer fitted with a thermo-regulation jacket. The temperature was adjusted to 5° C. A suitable cooling medium was pumped around the thermo-regulation jacket using a suitable electronically controlled thermo-regulation unit set at a suitable temperature in order to maintain 5°C within the main crystallizer vessel. The n-heptane non-solvent was pumped from the main crystallizer vessel around an external recirculation loop using a diaphragm pump operating at 20 L/h, and through a 60 ml thermo-regulated glass ultrasonic flow-cell fitted with a 20 kHz ultrasonic probe, prior to return to the main vessel.

The flow cell was thermo-regulated at 5°C.

Continuous ultrasound was applied at 30 W power.

A 1.5% w/v solution of carmoterol hydrochloride in methanol:ethanol 3:1 v/v was prepared and then pumped into the ultrasonic flow cell at a rate of 50 ml/h. Upon complete addition of the solution, the temperature of the emulsion was slowly raised to 50°C to effect removal of methanol from the droplets.

After 15 minutes, material started crystallizing out of the process liquor. The product was kept under suspension for further two hours under applied ultrasound at 50°C to remove most of the alcoholic solvents.

The microcrystalline product was isolated by supercritical carbon dioxide extraction of the n-heptane from the suspension.

### Example 2 - Characterisation of the particles of Example 1

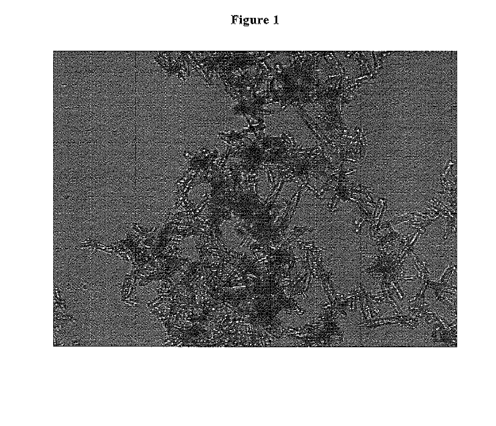

Figure 1 shows a SEM image of the particles obtained in Example 1 from which it can be clearly observed that said particles exhibit needle habit.

The obtained particles were characterised by differential scanning calorimetry (DSC).

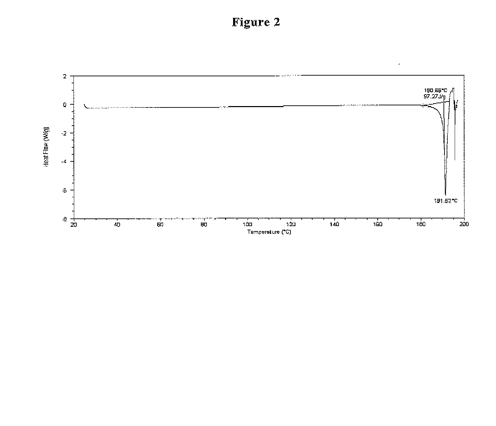

The thermogram, reported in Figure 2, shows the characteristic endothermic transition at approximately 191°C.

The slightly higher melting point recorded in comparison with carmoterol hydrochloride obtained according to the teaching of WO 2005/089760 is indicative of a higher purity of the material of Example 1. The heat of fusion value, ΔH_{f}, also confirms the higher purity (97.72 J/g *vs* 80.42 J/g).

The obtained particles were also characterised in terms of particle size distribution. The particle size has been determined by laser diffraction using a Mastersize X apparatus. The parameters taken into consideration are the volume diameters (VD) in micron of 10%, 50% and 90% of the particles expressed as d(v,0.1), d(v, 0.5) and d(v, 0.9), respectively, which correspond to the mass diameter assuming a size independent density for the particles. The mean values of eight samples are reported in Table 1. The standard deviation (S.D.) turns out to be less than ± 0.2.

**Table 1 - Particle size distribution**

| **Particle size (µm)** | **Particles of Example 1** | |
|---|---|---|
| | d(v, 0.1 ) | 1.91 |
| | d(v, 0.5) | 5.67 |
| | d(v, 0.9) | 14.03 |

### Example 3 - "Interactive ordered mixture" formulation comprising the particles of Example 1

The particles as obtained in Example 1 were added to a carrier prepared according to the teaching of EP 1274406 and reported hereafter.
a) Preparation of the fraction of the pharmacologically-inert microparticles.
   α -lactose monohydrate SpheroLac™ 100 with a starting mass diameter of 50 to 400 micron (MMD of about 170 micron) and magnesium stearate particles in the ratio 98:2 percent by weight were co-milled in a jet mill apparatus until the MMD of the whole mixture is less than 15 micron.
b) Addition of the fraction of microparticles to the fraction of coarse particles.
   90 percent by weight of α-lactose monohydrate CapsuLac™ (212 - 355 micron) was placed in a 240 ml stainless steel container, then 10 percent by weight of the fraction of pharmacologically-inert microparticles was added. The blend was mixed in a Turbula mixer for 2 hours at 42 r.p.m. to obtain the carrier.
c) Addition of the particles of Example 1 to the carrier.

The particles were added to the carrier in a suitable amount in order to obtain a ratio of 1 µg of carmoterol hydrochloride to 10 mg of final formulation. The resulting blend is mixed in a Turbula mixer for 30 min at 46 r.p.m.

### Example 4 - Characterisation of the powder formulation of Example 3

The powder formulation of Example 3 was characterised in terms of the uniformity of distribution of the active ingredient and aerosol performances after loading it in the multidose dry powder inhaler Pulvinal^{™}.

The uniformity of distribution of the active ingredients was evaluated by withdrawing six samples from different parts of the blend and evaluated by HPLC.

The evaluation of the aerosol performance was carried out using the Andersen Cascade Impactor (Apparatus D) according to the conditions reported in the European Pharmacopeia 6th Ed 2008, par 2.9.18, pages 293-295.

After aerosolization of 10 doses, the ACI apparatus was disassembled and the amounts of drug deposited in the stages were recovered by washing with a solvent mixture and then quantified by High-Performance Liquid Chromatography (HPLC). The following parameters, were calculated: *i)* the delivered dose which is the amount of drug delivered from the device recovered in the impactor; *ii)* the fine particle dose (FPD) which is the amount of delivered dose recovered in the S3-AF stages having a particle size equal to or lower than 5.0 micron; *iii)* the fine particle fraction (FPF) which is the percentage of the fine particle dose; iv) the MMAD.

The results in terms of uniformity of distribution and aerosol performances (mean value ± S.D) are reported in Table 2.

**Table 2 - Uniformity of distribution of the active ingredient and aerosol performances**

| | |
|---|---|
| | |
| Uniformity of distribution of the active ingredients (µg) | 0.99 ± 0.02 |
| Delivered dose (µg) | 0.97 ± 0.03 |
| Fine particle dose (FPD, µg) | 0.20 ± 0.04 |
| Fine particle fraction (FPF, %) | 23.3 ± 2.90 |
| MMAD (µm) | 2.86 ± 0.46 |
| | |

The particles of Example 1 disperse very well and show an excellent uniformity of distribution as it can be appreciated by the founded assay (99% of the theoretical value).

The relative standard deviation (RSD) turned out to be of about 2%.

Finally, the formulation shows acceptable aerosol performances in terms of respirable fraction.

## Claims

1. A process for preparing crystalline particles of a pharmaceutically acceptable salt of 8-hydroxy-5-[(1R)-1-hydroxy-2-[[(1R)-2-(4-methoxyphenyl)-1-methylethyl] amino]ethyl]-2(1H)-quinolinone (carmoterol), said process comprising the steps of:
a) preparing a solution of said active ingredient in a pre-determined ratio in a solvent;
b) contacting said solution in a first flowing stream with a non-solvent in a second flowing stream so as to form an emulsion;
c) applying high intensity ultrasound to induce crystallisation of the active ingredient; and
d) isolating and collecting the active ingredient particles,
wherein the solvent is a methanol:ethanol mixture in a ratio between 4:1 and 2:1 (v/v), and the anti-solvent is n-heptane.

2. The process as claimed in claim 1, wherein the methanol:ethanol ratio is 3:1 (v/v).

3. The process as claimed in claim 1 or 2, wherein the salt is selected from the group consisting of hydrochloride, hydrobromide, sulphate, phosphate, maleate, fumarate, tartrate, citrate, benzoate, mesylate, ascorbate, salicylate, acetate, succinate, lactate, glutarate or gluconate.

4. The process as claimed in claim 3, wherein the salt is the hydrochloride.

5. The process as claimed in any one of claims 1 to 4, wherein the ratio of the second flowing stream to the first flowing stream is comprised between 1000:1 to 1:1.

6. Crystalline microparticles of a pharmaceutically acceptable salt of 8-hydroxy-5-[(1R)-1-hydroxy-2-[[(1 R)-2-(4-methoxyphenyl)-1-methylethyl]amino]ethyl]-2(1H)-quinolinone (carmoterol) in form of needles obtained by the process of any one of claims 1 to 5.

7. A pharmaceutical formulation for administration by inhalation comprising the particles of claim 6, optionally together with one or more pharmaceutically acceptable excipients.

8. The formulation according to claim 7 in the form of dry inhalation powder.

9. A dry powder inhaler filled with the dry powder formulation according to claim 8.

10. The formulation according to claim 7 in the form of a suspension of the particles in a propellant.

11. A pressurized metered dose inhaler comprising a canister filled with the formulation of claim 10 and a metering valve for delivering a daily therapeutically effective dose of the formulation.
